# EUROPEAN PATENT APPLICATION

(11) **EP 0 672 419 A1**
(43) Date of publication of application: **20.09.1995**
(21) Application number: 92924890.4
(22) Date of filing: 08.12.1992
(51) Int. Cl.: A61K 37/30, A61K 9/08, C07K 7/36

(54) **BLOOD PRESSURE REGULATOR**

(30) Priority: 12.12.1991 JP 329065/91; 02.12.1992 JP 323445/92
(71) Applicant: Asahi Kasei Kogyo Kabushiki Kaisha, Osaka-shi Osaka 530 (JP)
(72) Inventor: SHIRAI, Takashi 541-36, Shimotogari Nagaizumicho, Shizuoka 411 (JP); MATSUMOTO, Kazuhiko 955-500, Kamisawa Kannamicho, Shizuoka 419-01 (JP); TAKEDA, Shohei 18-1209, Wakabadai 2-chome, Kanagawa 241 (JP)
(74) Representative: Woods, Geoffrey Corlett
(86) International application number: JP9201600
(87) International publication number: WO9311787

(57) **Abstract**

An agent for reducing blood pressure or maintaining a reduced blood pressure in hypotensive anesthesia or a hypotensive for treating anomalous hypertension occurring during surgical operations, containing a calcitonin gene related peptide (CGRP), a derivative thereof or a salt thereof as an active ingredient. When administered via intravenous drip under anesthetization, the active ingredient is short-acting, regulates the pressure rapidly, and serves to maintain cardiac functions without increasing the cardiac output.

## Description

### FIELD OF THE INVENTION

The present invention relates to an agent for reducing blood pressure or maintaining a reduced blood pressure in induced hypotension or a hypotensive agent for treating anomelous hypertension occuring during surgical operations, containing a calcitonin gene related peptide (CGRP), a derivative thereof or a salt thereof as an active ingredient.

### PRIOR ARTS

Human α or β type, rat α or β type, porcine type or chicken type of CGRP has already been known and its amino acid sequence consisting of 37 amino acids has been determined.

The amino acid sequence in animal apecies has been well conserved. human CGRP (hereinafter sometimes designates as h-CGRP) has been known a peptide which acts on hone metabolism and central nervous system. [Nature, 308(19), 746-748 (1984), FEBS Letters, 183(2), 403 (1985), Neuropeptides, 4, 425-435 (1984)and Nature, 313(3), 54-56 (1984)]
porcine CGRP (hereinafter sometimes designates as p-CGRP) has known as a peptide possessing increasing activity of heart rate. [Neuropeptides, 9, 75-82(1987)]. Rat CGRP (hereinafter sometimes designates as r-CGRP) has known as a peptide possessing vasodelating activity and suppressive action of gastric juice secretion. [British J. Pharmacol., 86, 544 (1985), Regulatory Peptides, 12, 81-89 (1985)]
CGRP exhibits vasodilating activity at f-molar order (10⁻¹⁵mole) and is called the strongest vasodilator [Nature, 313, 54 (1985)]. Therefore a study on vasodilator is in progress. Since h-CGRP seems to have utility for treating a deficient cerebral blood flow supply (Japan. Pat. Unexam. Publ., No. 2-502011), clinical cases for improving postoperative treatment of subarachnoid hemorrhage. [Lancet, 335, 869 (1990)] In all the cases a fall of blood pressure and increase in heart rate were observed, so that such an adverse action is overcome by lowering an amount of administration below 0.6 n mol/hour. (approx 4.6 ng/kg/hour) (PCT Pat. Unexam. Publ. No. 2-502011). A combination treatment with calcium antagonist is also reported (PCT Pat. Unexam. Publ, No. 3-505462).

Further administration of h-CGRP in canine is reported to lower blood pressure with increase in heart rate. [Am. J. Physiol., 257, R726-R731 (1989)]
Moreover, h-CGRP derivative, chicken CGRP (hereinafter sometimes designates as chicken CGRP) and its derivative has been hnown as a peptide having sea calcium and phosphorous reducing activities. (Japan. Pat. Unexam. Publ., No. 62-129297, ibid., No. 63-126814, ibid., No., 63-258490 and ibid., No. 64-26598)
Amino acid sequence of the above CGRP is shown in the following. 
1
H－A－
S－－－－－－－－－－－－S
2　3　4　5　6　7
－Cys－B－Thr－Ala－Thr－Cys
8　9　10　11　12
－Val－Thr－His－Arg－Leu
13　14　15　16　17　18
－Ala－C－D－Leu－Ser－Arg
19　20　21　22　23　24
－Ser－Gly－Gly－E－F－Lys
25　26　27　28　29　30
－G－Asn－Phe－Val－Pro－Thr
31　32　33　34　35　36
－I－Val－Gly－Ser－J－Ala
37
－Phe－NH₂
h-α-CGRP [A=Ala, B=Asp, C=Gly, D=Leu, E=Val, F=Val, G=Asn, I=Asn, J=Lys]
h-β-CGRP [A=Ala, B=Asn, C=Gly, D=Leu, E=Met, F=Val, G=Ser, I=Asn, J=Lys)
r-α-CGRP [A=Ser, B=Asn, C=Gly, D=Leu, E=Val, F=Val, G=Asp, I=Asn, J=Glu]
r-β-CGRP [A=Ser, B=Asn, C=Gly, D=Leu, E=Val, F=Val, G=Asp, I=Asn, J=Lys]
p-CGRP [A=Ser, B=Asn, C=Gly, D=Leu, E=Met, F=Val, G=Ser, I=Asp, J=Glu]
c-CGRP [A=Ala, B=Asn, C=Asp, D=Phe, E=Val, F=Gly, G=Asn, I=Asn, J=Lys]

### PROBLEMS TO BE SOLVED BY THE INVENTION

As illustrated, CGRP has various biological properties and hence to find out new pharmacological activity is still important for developing pharmaceuticals.

### MEANS FOR SOLVING THE PROBLEMS

In clinical cases of induced hypotension, every known hypotensive drugs can not always be applicable. Among clinically applied hypotensives, for example, hypotensive diuretic, sympatholytic agent, calcium antagonist has known. Pharmaceuticals used in induced hypotension are required to be administered intravenously with short term acting, controllable and lowering blood pressure without suppressing cardiac function and with maintaining blood flow in tissue. For these purposes in the clinical trials at hypotensive anesthesia, at present trimethaphan, nitroprusside, ATP, nitroglycerin, prostaglandin E₁, diltiazem hydrochloride and nicardipine have been applied. Among them, triamethaphan shows autonomic nerve blocking action and reduces cardiac output with suppressing release of renin and catecholamine, accordingly it suppress cardiac and renal functions and causes tachyphylaxis. Nitroprusside shows prolonged hypotension and activation of renin-angiotensin sympathetic nerve system, accordingly it causes hyper resistance during operation and rebound hypertension, further cyanide poisoning caused by its metabolitee is a critical defect. ATP has disadvantage with A-V block or metabolic acidosis.

A site of action of nitroglycerin is a capacitive vessel and so venous bleeding is diadvantageous. Prostaglandin E₁ induces phlebitis. Calcium channel blocking drug such as diltiazem and nicardipin shows activations of renin-angiotensin system and sympathetic nerve system and excessive hemodynamics. Those compounds are not satisfactory for the drugs used in induced hypotension.

Strong vasodilating and hypotensive activities of CGRP has known. However since CGRP has hypotensive activity together with strong increasing activity of heart rate which loads the heart heavily, so that it has been thought impossible to use in induced hypotension. For example, when h-CGRP is administered at 0.6 n mol/hour (approx. 4.6 ng/kg/hour) in human, blood pressure decreases but heart rate increses up extremely. (PCT WO 90/12815)

An unexpected discovery was made during the study on suppressing heart rate increasing activity of CGRP that prolonged intraveneous administration of CGRP in anesthesia is found to show various advantages, for example suppression of heart rate increase, maintaining cardiac functions, short time acting, rapid regulation of the pressure and continuous tissue blood flow, in the induced hypotension or the anomalous hypertension occuring during surgical operations.

An object of the present invention is to provide an agent for reducing blood pressure or maintaining a reduced blood pressure in induced anesthesia or a hypotensive for treating anomalous hypertension occuring during surgical operations, containing CGRP, a derivative thereof or a salt thereof as an active ingredient.

Examples of anesthetic used in the above induced hypotension are halothane, isoflurane, enflurane and desflurane.

Examples of an active ingredient of the present invention is CGRP, a derivative thereof or a salt thereof. CGRP and the derivative thereof can be synthesized by a known peptide synthesis such as liquid phase method or solid phase method. Examples of CGRP are h-α-CGRP, h-β-CGRP, c-CGRP, r-α-CGRP, r-β-CGRP and p-CGRP.

Examples of CGRP derivative are desalanyl-deamino-h-α-CGRP, desalanyl-deamino-h-β-CGRP, desalanyl-[Asu^{2,7}]-h-α-CGRP, desalanyl-[Asu^{2,7}] -h-β-CGRP, [Asn³,Phe¹⁵, Gly²³]-h-α-CGRP, desalanyl-deamino-[Asn³,Phe¹⁵, Gly²³]-h-α-CGRP, [Asn³,Asp¹⁴, Gly²³]-h-α-CGRP, desalanyl-deamino-[Asn³, Asp¹⁴, Gly²³]-h-α-CGRP, [Asn³,Asp¹⁴, Phe¹⁵]-h-α-CGRP, desalanyl-deamino-[Asn³,Asp¹⁴, Phe¹⁵]-h-α-CGRP,[Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³,Glu¹⁴, Gly²³]-h-α-CGRP, [Asn³,Glu¹⁴, Phe¹⁵]-h-α-CGRP, desalanyl-deamino-[Asn³, Glu¹⁴, Phe¹⁵]-h-α-CGRP, [Glu¹⁴]-h-α-CGRP, desalanyl-deamino-[Glu¹⁴]-h-α -CGRP, desalanyl-[Asu^{2,7}]-c-CGRP, desalanyl-[Asp³,Asu^{2,7}]-c-CGRP and desalanyl-deamino-c-CGRP.

A salt thereof can be a pharmacologically non-toxic salt. Examples of a salt are inorganic salt such as hydrochloride or sulfate and organic salt such as acetate, tartrate, succinate or malate.

Parenteral administration of the active ingredient of the present invention is preferable. For example, it can be administered intraveneously. Dosage can be varied according to a nature of the active ingredient, and is, generally in parenteral administration, a single dose of 1 µg - 200mg in adult. No death was observed when the active ingredient dissolved in 0.1M aqueous sodim acetate containing 0.1% bovine serum albumin is administered at 80 µg/kg body weight to Wistar rat, body weight 80-90g, through caudal vein.

The preparation of the present invention is preferably injectable form, but is not limited thereto. The preparation can be formulated a preferable dosage form.

A dosage form for injection can be prepared by dissolving the active ingredient with buffer agent, tonicity agent, pH adjuster and stabilizing agent in distilled water for injection, sterilizing through aspetic filter and pouring in an ampule, or by dissolving the active ingredient with extending agent and stabilizing agent in distilled water for injection, sterilizing through aseptic filter, pouring in a vial and lyophilizing.

The preparation of the present invention is also administered by intraveneous drip infusion for reducing blood pressure or maintaining a reduced blood pressure in hypotensive anesthesia. Further the preparation of the present invention can be administered at an surgical operations for a patient who has a complication with hypertension, ischemic heart disease, cerebrovascular disease or renal disorder. The induced hypotension herein referred means artificially reducing a blood pressure under anesthesia for the purpose of simplifying surgical operation or minimizing bleeding in an operation, and such the anesthesia has offen been applied in the operations.

Accordingly, an agent for reducing blood pressure or maintaining a reduced blood pressure in induced hypotension means the agent having a property to maintain cardiac function without increasing substantial heart rate and to maintain a reduced blood pressure.

Administering the acvtive ingredient of the present invention, a blood pressure reduces rapidly, and stopping the administration after surgical operation, normal blood pressure is recovered rapidly, so that artificail reduction of blood pressure and maintenance of reducing blood pressure in the anesthesia can be achieved. Further when the active ingredient of the present invention is administered at an anomalous hypertension occuring during surgical operations, such the anomalous blood pressure is recovered rapidly to normal pressure. Preferable administraion is an intraveneous drip infusion.

### EFFECT OF THE INVENTION

When administered the active ingredient of the present invention via intraveneous drip infusion under anesthetization, the active ingredient is short acting, regulates the pressure rapidly and serves to maintain cardiac functions without increasing the cardiac output. It provides idealistic aciton in induced hypotension and for an anomelous hypertension occuring during surgical operation, and can be applied for the surgical operation with safely to a patient who has a complication with hypertension, ischemic heart disease, cerebrovascular disease or renal disorder. Further, when the active ingredient of the present invention is administered under anesthesization, blood pressure reduces rapidly and is turned to recover normal blood pressure by stopping administration of the drug after surgical operation, so that it is a useful drug for artificially reducing blood pressure or maintaining reduced blood pressure.

### EXAMPLES

Following examples illustrate the preparation of the present invention.

### Example 1

Human-α-CGRP 60mg, mannitol 10g and preferable amount of stabilizing agent were dissolved in distilled water for injection 2 lit., passed through aseptic filter, filled each 1 ml to a vial and lyophilized to obtain preparation for injections of h-α-CGRP 30 µg/vial.

This preparation can be administered by dissolving with physiological saline before use.

### Example 2

Human-α-CGRP 60mg, buffer solution, isotonic solution, pH adjuster and stabilizing agent were dissolved in distilled water for injection 6 lit., and passed through an aseptic filter, filled each 1 ml to an ampule and sealed the ampule to obtain injectable form of h-α-CGRP 10 µg/ampule.

### Example 3

Desalanyl-deamino-c-CGRP 60mg, mannitol 10g and preferable amount of stabilizing agent were dissolved in distilled water for injection 2 lit., passed through aseptic filter, filled each 2 ml to a vial and lyophilized to obtain preparation for injections of desalanyl-deamino-c-CGRP 60 µg/vial.

This preparation can be administered by dissolving with physiological saline before use.

The other injectable preparation of CGRP derivative can be prepared by the same procedures.

### Example 4

Desalanyl-deamino-c-CGRP 60mg, buffer solution, isotonic solution, pH adjuster and stabilizing agent were dissolved in distilled water for injection 6 lit., and passed through an aseptic filter, filled each 1 ml to an ampule and sealed the ampule to obtain injectable form of desalanyl-deamino-c-CGRP 10 µg/ampule.

The other injectable preparation of CGRP derivative can be prepared by the same procedures.

Effects of the active ingredient of the present invention in hypotensive anesthesia are illustrated hereinbelow.

Abbreviations used herein are indicated as follows.
HR: Heart rate (beat/min)
CO: Cardiac output (1/min)
CI: Cardiac index (1/min/m²)
SV: Stroke volumes (ml/HR)
SI: Stroke index (ml/HR/m²)
CVP: Central venous pressure (mmHg)
SPAP: Systolic pulmonary artery pressure (mmHg)
DPAP: Diastolic pulmonary artery pressure (mmHg)
MPAP: Mean pulmonary artery pressure (mmHg)
PCWP: Pulmonary capillary wedge pressure (mmHg)
SAP: Systolic artery pressure (mmHg)
DAP: Diastolic artery pressure (mmHg)
MAP: Mean artery pressure (mmHg)
SVR: Systemic vascular resistance (dynes · sec/cm⁵)
PVR: Pulmonary vascular resistance (dynes · sec/cm⁵)
RPP: Rate pressure products (mmHg.beat/min)
CPP: Coronary perfusion pressure (mmHg)
LVP: Left ventricle pressure (mmHg)
LVEP: Left ventricle end pressure (mmHg)
LV dp/dt max: maximum left ventricular systolic ratio (mmHg/sec)
EtCO₂: Expiration terminal CO₂ pressure (mmHg)
LVSWI: Left ventricle stroke work index (g.m/beat/m²)
RVSWI: Right ventricle stroke work index (g.m/beat/m²)

### Example 5

### 1) Method

Adult mongrel dog, mean body weight 12.7 ± 3.1 kg, was anesthesized with pentobarbital 25 mg/kg, intratarcheally cannulated and maintained anesthetic condition by 0.87% halothane-oxygen (1 MAC, minimum anesthetic concentration). Respiration was controlled, by using respirator (Harvard Corp., Model 613E ) at Paco₂ 35±5 mmHg.

Catheters were introduced to right femoral vein and artery for monitering arterial blood pressure, arterial blood gas analysis and infusion, and left cardiac catheter was introduced to left femoral veine to monitor left ventricular pressure. The changes in left ventricular pressure were differentiated continuously using a pressure processor (Nihon Koden Co., EQ-601G) to measure a maximum left ventricular systolic ratio (LV dp/dt max). The active ingredient of the present invention was administered to left femoral vein. Pulmonary arterial catheter (7F, Swan-Ganz® catheter) was introduced through right external jugular vein to monitor pulmonary artery pressure (PAP), paulmonary arterial wedge pressure (pulmonary capillary wedge pressure, PCWP) and cardiac output (CO). Pressure was monitored by using disporsable pressure monitoring kit (Gould Corp.), and electrocardiogram, arteriala pressure, left ventricle pressure (LVP) and maximum left ventricular systolic ratio (LV dp/dt max) were monitored by polygraph (Nihon Koden Co.) . Cardiac output (CO) was measured by thermal dilution method using cardiac output monitor (Nihon Koden Co Model MTC6210). Blood gas analysis was made by using automatic blood gas analyser (Radiometer Corp. ABL3). Mean artery pressure (MAP), cardiac index(CI) stroke volume index (SI), systemic vascular resistance (SVR), pulmonary vascular resistance (PVR), left ventricle stroke work index (LVSWI) and coronary perfusion pressure (CPP) were calculated by standard calculation equations, respectively.

To maintain low blood pressure, the active ingredient was intraveneously administered for 60 minutes with maintaining maximum dose at 40 µg/kg and adjusting to maintain mean arteraial pressure at 50 mmHg. Physiological saline was used for maintenance infusion and administered in combination with the active ingredient at 7 ml/kg/hr. At blood sampling two fold volume of hydroxyethylstarch (6-HES) was supplementary administered. Body temperature was maintained at 37 ± 1°C by covering with blanket.

A value at time are than 60 minutes passed after completing the start of the experiment and stabilizing a depth of anesthesia and blood flow, is set as control (S₁). Monitorings were performed at 5 min., 30 min. and 60 min. under reduced blood pressure in induced hypotension, and at 10 min., 30 min. and 60 min. after terminating the reduced blood pressure in induced hypotension (after stopping administration), for totally seven points of time.

Results of the experiments using desalanyl-deamino-chicken CGRP is used as the active ingredient are shown in Tables 1, 2 and 3.

### 2) Results

**Table 1**

| | before administration | after administration | | | after terminating administration | | |
|---|---|---|---|---|---|---|---|
| | | 5min. | 30min. | 60min. | 10min. | 30min. | 60min. |
| HR | 148 | 143 | 132 | 138 | 136 | 138 | 134 |
| CO | 3.47 | 3.67 | 3.40 | 3.75 | 3.33 | 3.14 | 3.07 |
| CI | 4.36 | 4.65 | 4.24 | 4.95 | 4.33 | 4.07 | 4.14 |
| SV | 21.8 | 24.0 | 23.8 | 26.5 | 23.6 | 21.8 | 22.9 |
| SI | 29.5 | 32.5 | 32.2 | 35.9 | 31.9 | 29.5 | 30.9 |
| CVP | 5 | 4 | 4 | 4 | 4 | 4 | 6 |
| SPAP | 27 | 29 | 24 | 27 | 26 | 25 | 26 |

**Table 2**

| | before administration | after administration | | | after terminating administration | | |
|---|---|---|---|---|---|---|---|
| | | 5min. | 30min. | 60min. | 10min. | 30min. | 60min. |
| DPAP | 9 | 7 | 5 | 7 | 7 | 8 | 8 |
| MPAP | 16 | 16 | 12 | 15 | 15 | 15 | 16 |
| PCWP | 9 | 5 | 5 | 6 | 8 | 7 | 7 |
| SAP | 133 | 93 | 58 | 58 | 68 | 79 | 102 |
| DAP | 89 | 50 | 34 | 43 | 49 | 53 | 61 |
| MAP | 103 | 65 | 50 | 50 | 58 | 66 | 95 |
| SVR | 2420 | 1410 | 1160 | 1090 | 1340 | 1640 | 1950 |
| PVR | 173 | 255 | 177 | 196 | 174 | 211 | 234 |

**Table 3**

| | before administration | after administration | | | after terminating administration | | |
|---|---|---|---|---|---|---|---|
| | | 5min. | 30min. | 60min. | 10min. | 30min. | 60min. |
| LVSWI | 37.3 | 26.5 | 19.7 | 23.4 | 21.7 | 23.7 | 31.1 |
| RVSWI | 4.41 | 5.30 | 3.50 | 5.37 | 4.77 | 4.42 | 4.21 |
| RPP | 19600 | 13200 | 7650 | 8000 | 9240 | 10900 | 13600 |
| CPP | 80 | 45 | 29 | 37 | 41 | 46 | 54 |
| LVP | 132 | 102 | 86 | 95 | 110 | 110 | 111 |
| LVEP | 1 | 0 | 0 | 0 | 0 | 0 | 0 |
| LVdp/dt max | 3000 | 3000 | 2600 | 2600 | 3000 | 3000 | 3000 |
| ETCO₂ | 38 | 37 | 35 | 34 | 32 | 30 | 30 |

As shown in the above, desalanyl-deamino-c-CGRP shows reducing blood pressure without increasing heart rate and with maintaining cardiac function. Accordingly it is useful for reducing blood pressure or maintaining a reduced blood pressure in induced hypotension.

### Example 6

Example 5 was repeated by substantially almost same manner using ten adult mongrel dogs, mean body weight 14.7±2.1 kg, applying same anesthetic condition with maintaining artificially hypotensive with average arterial blood pressure at 60 mmHg for 60 min., and administering the active ingredient desalanyl-deamino-c-CGRP.

A value at time more than 60 minutes passed after completing the start of the experiment and stabilizing a depth of anesthesia and blood flow, was set as control (S₁). Monitorings were performed at 30 min. and 60 min. under reduced blood pressure in induced hypotension, and at 10 min. and 30 min. after terminating the reduced blood pressure in induced hypotension (after stopping administration), for totally five points of time.

Results are shown by mean ± S.D.

Amount of the active ingredient for maintaining average arterial blood pressure at 60 mmHg for 60 minutes was 5 µg-115 µg/kg. Results are shown in Tables 4 and 5.

**Table 4**

| | reduced blood pressure 0 min. | reduced blood pressure 30 min. | reduced blood pressure 60 min. |
|---|---|---|---|
| HR | 155.7 ± 29.6 | 149.3±31.7 | 154.3 ± 33.8 |
| CO | 2.08 ±0.683 | 2.348±0.528 | 2.739 ±0.518 * |
| CI | 2.928±0.854 | 3.418±0.692 | 4.006 ±0.771 ** |
| SV | 13.588±5.573 | 16.485±5.783 | 18.510 ±5.942 |
| SI | 19.461±6.806 | 23.810±6.952 | 27.030 ±7.664 |
| CVP | 2.5 ±1.2 | 2.5±1.1 | 2.2±1.2 |
| SPAP | 22.8 ±6.8 | 23.0±6.2 | 25.5±7.3 |
| DPAP | 10.8 ±4.7 | 9.9±4.0 | 11.3±4.9 |
| MPAP | 16.2 ±5.7 | 15.6±5.3 | 17.7±5.9 |
| PCWP | 10.1 ±4.1 | 9.0±4.0 | 9.4±4.7 |
| SAP | 152.7 ±19.9 | 76.5±4.8 ** | 74.4±4.9 ** |
| DAP | 94.0 ±11.7 | 47.3±2.8 ** | 47.7±2.6 ** |
| MAP | 113.7±12.8 | 60.4±0.7 ** | 60.1±0.9 ** |
| SVR | 4839.0±1634.6 | 2054.0±455.6 ** | 1748.0±409.1 ** |
| PVR | 268.06±137.84 | 231.10±80.62 | 251.20±97.14 |
| LVSWI | 27.060±8.641 | 16.906±5.750 ** | 18.949±6.280 ** |
| RVSWI | 3.429±1.428 | 3.979±1.239 | 5.380±1.747 ** |
| RPP | 23890.0±6528.1 | 11357.0±2260.5** | 11462.0±2765.1** |
| CPP | 83.9±12.3 | 38.3±4.5 ** | 38.3±4.9 ** |
| LVP | 149.1±20.7 | 102.7±14.1** | 104.7±16.3** |
| LVEP | 0.3±0.7 | 0.0±0.0 | 0.0±0.0 |
| LVdp/dt max | 3200.0±639.4 | 3070.0±771.8 | 3210.0±749.0 |
| ETCO2 | 38.8±1.8 | 38.5±5.6 | 39.8±6.9 |

| | | | |
|---|---|---|---|
| Mean ± S.D * : P = 0.05 | | | |
| * * : P = 0.01 (Dunnet Test) | | | |

**Table 5**

| | after terminating reduced blood pressure 10 min. | after terminating reduced blood pressure 30 min. |
|---|---|---|
| HR | 158.8±33.8 | 151.6±27.9 |
| CO | 2.751±0.438 * | 2.487±0.378 |
| CI | 4.025±0.685 ** | 3.638±0.587 |
| SV | 18.170±5.632 | 17.030±5.071 |
| SI | 26.300±6.720 | 24.760±6.720 |
| CVP | 2.9±1.5 | 3.2±1.1 |
| SPAP | 26.8±7.2 | 27.4±7.2 |
| DPAP | 11.3±5.2 | 11.3±5.4 |
| MPAP | 18.3±6.0 | 18.7±6.3 |
| PCWP | 9.2±4.1 | 10.6±4.5 |
| SAP | 94.4±7.2 ** | 119.4±10.1** |
| DAP | 59.2±5.1 ** | 74.1±4.7 ** |
| MAP | 75.7±4.3 ** | 92.8±6.3 ** |
| SVR | 2162.0±419.2 ** | 2961.0±700.9 ** |
| PVR | 273.30±100.09 | 269.10±109.58 |
| LVSWI | 23.970±6.998 | 27.680±7.370 |
| RVSWI | 5.209±1.501 * | 4.882±1.138 |
| RPP | 14920.0±3480.0** | 18100.0±3938.4** |
| CPP | 50.5±5.5 ** | 63.5±5.3 ** |
| LVP | 119.5±16.3** | 132.3±20.0 |
| LVEP | 0.1±0.3 | 0.0±0.0 |
| LVdp/dt max | 3370.0±783.2 | 3220.0±708.4 |
| ETCO2 | 39.5±5.8 | 39.3±5.9 |

| | | |
|---|---|---|
| Mean ± S.D. * : P = 0.05 | | |
| * * : P = 0.01 (Dunnet Test) | | |

As shown in the above, desalanyl-deamino-c-CGRP shows reducing blood pressure without increasing heart rate and with maintaining cardiac function. Accordingly it is useful for reducing blood pressure or maintaining a reduced blood pressure in induced hypotension.

### Example 7

Example 6 was repeated by substantially almost same manner using eight adult mongrel dogs, mean body weight 11.3±2.3 kg, applying same anesthetic condition. Human α-CGRP was used as the active ingredient with administering at 10 µg/kg/hr and 30 µg/kg/hr, and heart rate (HR), mean artery pressure (MAP), left ventricle pressure (LVP) and maximum left ventricular systolic ratio (LV dp/dt max) were monitored.

A control value was set as 100% at more than 60 minutes after setting up the experimental start and confirming to stabilizing the depth of anesthesia and blood flow.

Monitoring were set up expressing with a value of per centage (%) for the control at 5 min., 10 min. and 30 min. after starting the administration, and at 5 min., 10 min., 30 min. and 60 min. after terminating the administration.

Results are shown in Tables 6 and 7.

In table 6, the experimental result upto 30 minutes after starting the adminsitration is illustrated, and in table 7, the test result from 60 minutes after starting the administration to 60 minutes after terminating the administration.

As illustrated in the results hereinabove, h-CGRP can be used as same as of desalanyl-deamino-c-CGRP, for reducing blood pressure or maintaining a reduced blood pressure in induced hypotension.

**Table 6**

| | amount of administ.(µg/kg/h) | before administ. (100 %) | after administ. | | |
|---|---|---|---|---|---|
| | | | 5 min. | 10 min. | 30 min |
| HR | 10 | 139.0±13.2 | 104.4±2.3 | 103.3±1.6 | 102.1 ±2.3 |
| | 30 | 145.5±13.1 | 98.3±5.4 | 97.8±5.1 | 98.8 ±7.2 |
| MAP | 10 | 109.3±2.4 | 62.3±2.5 | 56.5±1.3 | 52.0 ±2.2 |
| | 30 | 99.8±6.9 | 55.6±3.9 | 55.2±4.1 | 53.7 ±4.0 |
| LVP | 10 | 119.3±2.2 | 71.4±2.3 | 66.8±0.9 | 63.6 ±3.0 |
| | 30 | 110.8±7.1 | 65.4±2.8 | 64.7±3.0 | 63.5 ±3.6 |
| LVdp/dt max | 10 | 1730.0±115.8 | 92.8±6.5 | 81.8±4.3 | 77.6 ±3.2 |
| | 30 | 1887.5±83.6 | 69.6±5.9 | 68.0±5.8 | 67.6 ±6.2 |

**Table 7**

| | amount of admini. (µg/kg/h) | after administ. (%) 60 min. | after terminating administ.(%) | | |
|---|---|---|---|---|---|
| | | | 10 min. | 30 min. | 60 min. |
| HR | 10 | 108.0±3.5 | 107.7±3.9 | 104.5±4.4 | 101.4 ±2.9 |
| | 30 | 109.9±5.8 | 109.2±4.9 | 107.9±3.8 | 107.4 ±4.2 |
| MAP | 10 | 56.5±1.7 | 70.3±3.5 | 80.0±2.4 | 85.0 ±1.9 |
| | 30 | 61.2±2.5 | 66.4±4.2 | 77.4±3.5 | 83.3 ±3.3 |
| LVP | 10 | 68.2±2.9 | 79.1±3.6 | 87.0±1.9 | 90.7 ±1.9 |
| | 30 | 70.5±1.9 | 74.7±2.6 | 83.4±2.6 | 88.0 ±2.4 |
| LVdp/dt max | 10 | 88.3±5.1 | 101.2±9.3 | 107.3±8.6 | 100.8 ±6.2 |
| | 30 | 82.2±5.0 | 86.4±5.2 | 96.7±6.5 | 101.1 ±7.7 |

### Example 8

Example 6 was repeated by substantially almost same manner using four adult mongrel dogs, mean body weight 12.3±3.0 kg, applying same anesthetic condition. After setting up the experiment at more than 60 minutes passing and confirming to stabilize the depth of anesthesia and blood flow, phenylephrine (hypertensor) 10 µg/kg/min was administered.

Further after 10 minutes passed, the active ingredient, desalanyl-deamino-c-CGRP 10 µg/kg/hr was administered and at 10 min. and 20 min. after administration of the active ingredient, heart rate (HR) and mean a artery pressure (MAP) were measured. Result is shown in Table 8.

**Table 8**

| | before administ. | after administ. of phenylephrine | after administ. of CGRP | |
|---|---|---|---|---|
| | | 10 min. | 10 min(20min) | 20 min(30min) |
| HR | 133.0±10.3 | 138.0±9.3 | 102.7 +1.9 | 104±2.8 |
| MAP | 102.5±5.9 | 159.0±6.2 | 104.8 ±2.7 | 184±4.9 |

The result hereinabove shows that desalanyl-deamino-c-CGRP can be used for reducing blood pressure at anomalous hypertension occuring during surgical operations.

## Claims

1. An agent for reducing blood pressure or maintaining a reduced blood pressure in induced hypotension containing a calcitonin gene related peptide (CGRP), a derivative thereof or a salt thereof as an active ingredient.

2. An agent according to claim 1 wherein CGRP is human α-CGRP (h-α-CGRP), human β-CGRP(h-β-CGRP), chicken CGRP (c-CGRP), rat α-CGRP (r-α-CGRP), rat β-CGRP (r-β-CGRP) or porcine CGRP (p-CGRP).

3. An agent according to claim 1 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³,Asp¹⁴,Gly²³]-h-α-CGRP,desalanyl-deamino-[Asn³,Asp¹⁴, Gly²³]-h-α-CGRP, [Asn³, Asp¹⁴, Phe¹⁵]-h-α-CGRP,desalanyl-deamino-[Asn³,Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴,Gly²³]-h-α-CGRP, [Asn³,Glu¹⁴,Phe¹⁵]-h-α-CGRP, desalanyl-deamino- [Asn³,Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³,Asu^{2,7}]-c-CGRP and desalanyl-deamino-c-CGRP.

4. An agent according to cliams 1 - 3 wherein the said agent for reducing blood pressure or maintaining a reduced blood pressure is the agent which has an activity to maintain cardiac function without increasing the cardiac output and reduced blood pressure under anesthetic condition.

5. An agent according to cliam 4 wherein an amount of content of the active ingredient is an essential amout for controlling and maintaining cardiac function without increasing the cardiac output and reduced blood pressure under anesthetic condition.

6. An agent according to cliams 1 to 5 which is a preparation for drip infusion.

7. A use of CGRP, a derivative thereof or a salt thereof for producing pharmaceutical composition for applying reducing blood pressure or maintaining a reduced blood pressure in induced hypotension.

8. A use according to claim 7 wherein CGRP is h-α-CGRP, h-β-CGRP, c-CGRP, r-α-CGRP, r-β-CGRP or p-CGRP.

9. A use according to claim 7 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³,Asp¹⁴,Gly²³]-h-α-CGRP, desalanyl-deamino-[Asn³,Asp¹⁴, Gly²³]-h-α-CGRP, [Asn³, Asp¹⁴, Phe¹⁵]-h-α-CGRP,desalanyl-deamino-[Asn³,Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴,Gly²³]-h-α-CGRP, [Asn³,Glu¹⁴,Phe¹⁵]-h-α-CGRP, desalanyl-deamino- [Asn³,Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³,Asu^{2,7}]-c-CGRP and desalanyl-deamino-c-CGRP.

10. A use according to claims 7-9 wherein a pharceutical composition is a preparation for drip infusion.

11. CGRP, a derivative thereof or a salt thereof for applying reducing blood pressure or maintaining a reduced blood pressure in induced hypotension.

12. CGRP, a derivative thereof or a salt thereof for applying reducing blood pressure or maintaining a reduced blood pressure in induced hypotension having an action to maintain cardiac function without substantially increasing the cardiac output and reduced blood pressure.

13. CGRP, a derivative thereof or a salt thereof according to claims 11 and 12 wherein CGRP is h-α-CGRP, h-β-CGRP, c-CGRP, r-α-CGRP, r-β-CGRP or p-CGRP.

14. A derivative of CGRP or a salt thereof according to claims 11 and 12 wherein a derivative of CGRP is desalanyl-deamino-h-α-CGRP, desalanyl-desmino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³,Asp¹⁴,Gly²³]-h-α-CGRP, desalanyl-deamino-[Asn³, Asp¹⁴, Gly²³]-h-α-CGRP, [Asn³, Asp¹⁴, Phe¹⁵]-h-α-CGRP, desalanyl-deamino-[Asn³, Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴,Gly²³]-h-α-CGRP, [Asn³,Glu¹⁴,Phe¹⁵]-h-α-CGRP, desalanyl-deamino- [Asn³, Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.

15. A method for reducing blood pressure or maintaining a reduced blood pressure under anesthetic condition of humans which comprises administering a preparation containing effective amount of CGRP, a derivative thereof or a salt thereof under anesthetic condition in humans.

16. A method for reducing blood pressure or maintaining a reduced blood pressure under anesthetic condition of humans which comprises administering a preparation containing effective amount of CGRP, a derivative thereof or a salt thereof, the preparation of which is essential for controlling to maintain cardiac function without increasing the cardiac output and reduced blood pressure, under anesthetic condition in humans.

17. A method according to claims 15 and 16 wherein CGRP is h-α-CGRP, h-β-CGRP, c-CGRP, r-α-CGRP, r-β-CGRP or p-CGRP.

18. A method according to claims 15 and 16 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³,Asp¹⁴, Gly²³]-h-α-CGRP, desalanyl-deamino-[Asn³, Asp¹⁴, Gly²³]-h-α-CGRP, [Asn³, Asp¹⁴, Phe¹⁵]-h-α-CGRP, desalanyl-deamino-[Asn³,Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴, Gly²³]-h-α-CGRP, [Asn³, Glu¹⁴, Phe¹⁵]-h-α-CGRP, desalanyl-deamino- [Asn³, Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.

19. A method according to claims 15 - 18 wherein a preparation is the preparation for drip infusion.

20. An agent for reducing blood pressure for treating anomalous hypertension occuring surgical operations containing CGRP, a derivative thereof or a salt thereof as an active ingredient.

21. An agent according to claim 20 wherein CGRP is h-α-CGRP, h-β-CGRP, c-CGRP, r-α-CGRP, r-β-CGRP or p-CGRP.

22. An agent according to claim 20 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³, Asp¹⁴, Gly²³]-h-α-CGRP, desalanyl-deamino-[Asn³, Asp¹⁴, Gly²³]-h-α-CGRP, [Asn³, Asp¹⁴, Phe¹⁵]-h-α-CGRP, desalanyl-deamino-[Asn³, Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴, Gly²³]-h-α-CGRP, [Asn³, Glu¹⁴, Phe¹⁵]-h-α-CGRP, desalanyl-deamino- [Asn³, Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.

23. An agent for reducing blood pressure according to claims 20 - 22 wherein an amount of content of the active ingredient is an essential amout for controlling and maintaining cardiac function without increasing the cardiac output and reduced blood pressure under anesthetic condition.

24. An agent according to claims 20 - 23 wherein the agent is the prepartion for drip infusion.

25. A use of CGRP, a derivative thereof or a salt thereof for producing pharmaceutical composition for applying treatment of anomalous hypertension occurs during surgical operations.

26. A use according to claim 25 wherein CGRP is h-α-CGRP, h-β-CGRP, c-CGRP, r-α-CGRP, r-β-CGRP or p-CGRP.

27. A use according to claim 25 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³, Asp¹⁴, Gly²³]-h-α-CGRP, desalanyl-deamino-[Asn³, Asp¹⁴, Gly²³]-h-α-CGRP, [Asn³, Asp¹⁴, Phe¹⁵]-h-α-CGRP, desalanyl-deamino-[Asn³, Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴, Gly²³]-h-α-CGRP, [Asn³, Glu¹⁴,Phe¹⁵]-h-α-CGRP, desalanyl-deamino- [Asn³, Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.

28. A use according to claims 25-27 wherein a pharceutical composition is a preparation for drip infusion.

29. CGRP, a derivative thereof or a salt thereof for applying an agent for treating anomalous hypertension occuring during surgical operations.

30. CGRP, a derivative thereof or a salt thereof according to claim 29 wherein CGRP is h-α-CGRP, h-β-CGRP, c-CGRP, r-α-CGRP, r-β-CGRP or p-CGRP.

31. CGRP, a derivative thereof or a salt thereof according to claim 29 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³, Asp¹⁴, Gly²³]-h-α-CGRP, desalanyl-deamino-[Asn³, Asp¹⁴, Gly²³]-h-α-CGRP, [Asn³, Asp¹⁴, Phe¹⁵]-h-α-CGRP, desalanyl-deamino-[Asn³, Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴, Gly²³]-h-α-CGRP, [Asn³, Glu¹⁴, Phe¹⁵]-h-α-CGRP, desalanyl-deamino- [Asn³, Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.

32. A method for treating anomalous hypertension occuring during surgical operations of humans which comprises administering a preparation containing effective amount of CGRP, a derivative thereof or a salt thereof under anesthetic condition in humans.

33. A method for treating anomalous hypertension occuring during surgical operations of humans which comprises administering a preparation containing effective amount of CGRP, a derivative thereof or a salt thereof, the preparation of which is essential for controlling to maintain cardiac function without increasing the cardiac output and reduced blood pressure, under anesthetic condition in humans.

34. A method according to claims 32 and 33 wherein CGRP is h-α-CGRP, h-β-CGRP, c-CGRP, r-α-CGRP, r-β-CGRP or p-CGRP.

35. A method according to claims 32 and 33 wherein a derivative of CGRP is desalanyl-deamino-h-α-CGRP, desalanyl-deamino-h-β-CGRP, desalanyl-[Asu^{2,7}]-h-α-CGRP, desalanyl-[Asu^{2,7}]-h-β-CGRP, [Asn³, Phe¹⁵, Gly²³]-h-α-CGRP, desalanyl-deamino-[Asn³, Phe¹⁵, Gly²³]-h-α-CGRP, [Asn³,Asp¹⁴,Gly²³]-h-α-CGRP, desalanyl-deamino-[Asn³,Asp¹⁴, Gly²³]-h-α-CGRP, [Asn³, Asp¹⁴, Phe¹⁵]-h-α-CGRP, desalanyl-deamino-[Asn³,Asp¹⁴, Phe¹⁵]-h-α-CGRP, [Asp¹⁴]-h-α-CGRP, desalanyl-deamino-[Asp¹⁴]-h-α-CGRP, [Asn³, Glu¹⁴, Gly²³]-h-α-CGRP, desalanyl-deamino-[Asn³, Glu¹⁴,Gly²³]-h-α-CGRP, [Asn³, Glu¹⁴, Phe¹⁵]-h-α-CGRP, desalanyl-deamino- [Asn³,Glu¹⁴, Phe¹⁵]-h-α-CGRP, [Glu¹⁴]-h-α-CGRP, desalanyl-deamino-[Glu¹⁴]-h-α-CGRP, desalanyl-[Asu^{2,7}]-c-CGRP, desalanyl-[Asp³, Asu^{2,7}]-c-CGRP and desalanyl-deamino-c-CGRP.
